# EUROPEAN PATENT APPLICATION

(11) **EP 2 128 243 A1**
(43) Date of publication of application: **02.12.2009**
(21) Application number: 09160822.4
(22) Date of filing: 20.05.2009
(51) Int. Cl.: C12N 1/20, A61K 35/74

(54) **Novel strain of the genus Lactobacillus and topical pharmaceutical formulations containing it**

(30) Priority: 22.05.2008 IT MI20080949
(71) Applicant: Sinclair Pharma S.R.L., 20124 Milano (IT)
(72) Inventor: Mastrodonato, Marco, 20129 Milano (IT)
(74) Representative: Minoja, Fabrizio

(57) **Abstract**

This invention relates to a novel strain of lactobacillus, called *Lactobacillus acidophilus* EUVAG, and topical vaginal pharmaceutical formulations containing it, for use in all forms of dysbiosis and variations in vaginal pH which can cause recurrent bacterial or fungal vaginal infections.

## Description

The present invention relates to a novel strain of the genus *Lactobacillus,* and pharmaceutical formulations containing it.

More particularly, the present invention relates to a novel strain of *Lactobacillus acidophilus* of human origin, and topical vaginal formulations containing it, for use in all forms of dysbiosis and variations in vaginal pH which can cause recurrent bacterial or fungal vaginal infections.

The novel strain of *Lactobacillus acidophilus* was deposited with DSMZ (Deutsche Sammlung von Mikroorganismen und Zellkulturen GmbH) under number 19290 on 19 April 2007, according to the regulations of the Treaty of Budapest.

### BACKGROUND

In recent years, microbiological research has made considerable progress in discovering the main functions performed by the various germs in microbial ecosystems.

These studies have not only brought to light most of the bacterial species found in the intestinal lumen, for example (approx. 400), but have also demonstrated that some of them are useful in maintaining the stability and homeostasis of the entire ecosystem, as well as being involved in vitally important processes.

The vaginal microbiota also possesses a well-represented flora with conspicuous presence of lactic acid bacteria. The vagina, which is sterile at birth, is colonised by material lactobacilli within a few days. This agrees with the theory that normal human microbial flora derive mainly from the mother. In most cases, this flora will remain prevalent throughout life; in the vagina, its presence will be mainly influenced by hormone levels. The presence of lactobacilli is strongly influenced by the availability of glycogen, the levels of which are regulated by the oestrogens.

A few weeks after birth there is a reduction in the lactobacillus population, to the advantage of coagulase-negative staphylococci, streptococci, *Escherichia coli* and other intestinal bacteria. Later, at puberty, the ideal conditions for multiplication of lactobacilli are restored (increased oestrogen levels and thickening of the vaginal mucosa); lactobacilli thus become the dominant vaginal flora in adult women. During this period the microbial population in a vaginal secretion amounts to approx. 10⁷- 10⁸ CFU/g. This microbial population remains substantially unchanged until the menopause, when it is replaced by a mixed flora, not unlike that of the prepuberal period, but with a considerable presence of mycoplasma and, to a lesser extent, anaerobic bacteria (e.g. *Gardnerella vaginalis*)*.*

Various studies have attempted to characterise the vaginal lactobacillary flora at species level. These studies demonstrate that there is a geographical difference in the composition of the vaginal microflora. However, bacteria belonging to what is often called the *Lactobacillus acidophilus* complex seem to constitute the dominant flora at childbearing age.

These micro-organisms are considered by numerous authors to be the factors mainly responsible for the low physiological values of the vaginal pH. In fact the main, and in some cases the only product of the breakdown of glycogen performed by these micro-organisms is lactic acid. Consequently, the presence of lactobacilli allows the vaginal pH to be maintained at around the physiological values of 4.5 - 5.5.

The presence of lactobacilli as the dominant flora can therefore represent a useful benchmark which indirectly indicates the condition of the vaginal ecosystem.

Vaginal acidity at childbearing age not only affects the characteristics of the native microbiota with a selective mechanism in favour of acid-tolerant micro-organisms, but above all creates an environment hostile to colonisation by those arriving from the exterior. In fact, a reduction in vaginal lactobacilli in favour of mixed populations seems to be one of the main causal factors of vaginosis, and recurrent episodes are often associated with inability to restore the normal vaginal microflora rich in lactobacilli. The ability of the lactobacilli to produce hydrogen peroxide seems to play an equally important part in preventing the growth of bacteria responsible for vaginosis, such as *G. vaginalis* and *Prevotella bivia.*

Bacterial vaginosis, the most common disorder of the vagina, is characterised by an increase in pH and excessive growth of anaerobic bacteria. The increased pH is harmful to the survival of lactobacilli, which consequently become incapable of maintaining a normal balance in the vaginal ecosystem. The first method of restoring the correct composition of the vaginal microflora could be to restore the pH values to normal; lactic acid bacteria can be used for this purpose, as they produce quite large amounts of lactic acid or other acids during their metabolism.

Numerous "useful" lactic acid bacteria are currently used, in different types of formulations, such as euregulators and microflora supplements.

In this respect, particular attention has been paid to the use of lactobacilli, which seem to perform a far more important role in promoting and guaranteeing the health of the host than other micro-organisms also available on the market.

In fact, recent microbiological findings demonstrate that some species of lactobacilli possess a series of special characteristics which justify their use as diet products or as adjuvants during pharmacological treatment.

The lactobacilli of the vaginal and intestinal "fermenting flora" maintain the homeostasis of the microflora by producing lactic acid and bacteriocins or stimulating the immune system. They are also involved in a series of metabolic reactions of vital importance to the host: deconjugation of bile acids, breakdown of nitrosamine, vitamin synthesis, and metabolism of cholesterol and steroid hormones.

Several studies have also demonstrated that lactobacilli are involved in some way in the intricate mechanism of hormone synthesis.

An anticarcinogenic activity (lower risk of colon cancer in individuals who use milk enzymes) and the ability to deactivate drugs and bacterial toxins have recently been attributed to lactobacilli.

The contribution made by lactobacilli to maintaining a normal vaginal ecosystem probably involves multiple actions:
- adhering to the vaginal epithelium and interfering with the adherence, invasion, translocation and growth of potential pathogens;
- stimulating the release of anti-inflammatory cytokines which inhibit colonisation by pathogenic bacteria;
- reducing the pH values, thus promoting further growth of lactobacilli and preventing the growth of pathogens.

However, said characteristics would be of limited usefulness if lactobacilli did not possess "probiotic" properties which allow them to survive in hostile environments (resistance to extreme pH values) and colonise the epithelia (adherence and hydrophobicity).

Moreover, from the standpoint of industrial production, some "technological" characteristics of the bacteria are equally important, such as resistance to freeze-drying, growth rate and vitality over time, which guarantee a high-quality product.

There is consequently a need for lactobacilli which possess properties that guarantee stable colonisation of the vaginal ecosystem and perform a favourable regulation function in all conditions where its alteration leads to a pathological event.

### DESCRIPTION OF THE INVENTION

A bacterial strain belonging to the *Lactobacillaceae* family has now been found which presents euregulating properties in the vagina, and can therefore be used beneficially in all forms of dysbiosis and variations of vaginal pH which can cause recurrent bacterial or fungal vaginal infections.

The novel strain of *Lactobacillus acidophilus* was deposited with DSMZ (Deutsche Sammlung von Mikroorganismen und Zellkulturen GmbH) under number 19290 on 19 April 2007, according to the regulations of the Treaty of Budapest.

The present invention therefore relates to a novel lactobacillus, hereinafter called "lactobacillus EUVAG" or "EUVAG", and its therapeutic or prophylactic use in the treatment of alterations of the vaginal microflora or pH caused by lengthy antibiotic treatments, hormone imbalance or recurrent vaginal infections.

The EUVAG lactobacillus according to the invention possesses colonisation, adherence, resistance and growth rate characteristics superior to those of known strains.

The microbiological characteristics of *L. acidophilus* EUVAG are illustrated below and in Tables 1-6.
**Origin:** the vagina of healthy women
**Morphology**: Gram-positive sporeless bacilli
**Stability at acid pH**: YES
**Stability at basic pH:** YES
**Hydrophobic capacity (salting out):** YES
**Adherence to buccal cells:** YES
**Adherence to CaCo-2 cells**: YES
**Adherence to HeLa cells:** YES
**Growth rate:** high
**Resistance to freeze-drying:** 62% survival after freeze-drying
**Production of H₂O₂:** YES
**Production of lactic acid:** YES
PHARMACOLOGICAL TRIALS

The research was divided into the following stages:
I) Isolation of lactobacilli from healthy female volunteers;
II) Selection of strains with the characteristics considered most useful: high adherence, hydrophobicity, and resistance to adverse environmental conditions (extreme pH values);
III) Among the bacteria selected in point II, strains which also presented good "technological" characteristics (rate of reproduction, resistance to freeze-drying) were identified;
IV) The lactobacillus selected in the preceding stages was also characterised by the production of hydrogen peroxide and lactic acid, substances which can contribute to the antibacterial activity of some probiotics.

### PHASE I

### Isolation of lactobacilli

Vaginal swabs obtained from healthy women of childbearing age were evaluated from the microbiological standpoint. 50 "permanent" bacterial species belonging to the genus *Lactobacillus* were isolated from these samples.

The isolation was conducted in MRS agar (VWR International) after anaerobic incubation for 48 hours at 37°C.

All catalase-negative, lactic-acid-producing colonies whose bacterial morphology, on observation under the optical microscope, was attributable to that of Gram-positive sporeless bacilli, were considered to belong to the genus *Lactobacillus,* and consequently processed for identification.

The identification was performed with sugar fermentation tests using the API 50 CHL system (BioMerieux). This standardised system allows the study of the fermentation metabolism of carbohydrates and some derivatives (heterosides, polyalcohols and uronic acids) to be evaluated on the basis of a large number of biochemical tests. The micro-organism is inoculated into each test tube of the strip, followed by anaerobic incubation, during which catabolism of the carbohydrates produces organic acids that cause a variation in pH, detected by the indicator in the growth medium (bromocresol purple).

The fermentation profile of *L. acidophilus* EUVAG is illustrated in Table 1.

### PHASE II

### Adherence

The ability to adhere to epithelial cells is one of the pre-requisites for the use of enzymes as an adjuvant of treatment or for prophylactic purposes.

This characteristic enables the bacteria to anchor specifically to the epithelia, colonise them by multiplying, and consequently perform their metabolic and immunostimulation activities *in situ.*

Buccal cells and cells of the Caco-2 line (human colon tumour cells) and the HeLa line (human cervical tumour cells) were used to evaluate this parameter. The buccal cells were collected from healthy non-smoking volunteers, washed several times to eliminate any bacteria, and resuspended in PBS at the concentration of 10⁵ cells/ml. The Caco-2 and HeLa cells were grown until a homogenous single layer was obtained.

The lactobacilli were inoculated into MRS broth and incubated for 24 hours at 37°C in an atmosphere enriched with 5% CO₂.

To evaluate adherence, 10⁷ bacteria/ml were added to 10⁵ buccal cells/ml or to the single cell layer and incubated at 37°C for 60 min under continuous stirring.

At the end of the incubation period, after repeated washes to eliminate the non-adhering bacteria, the cells were placed on slides, which were dried in air, fixed with methanol, and stained with the Giemsa stain.

The bacteria adhering to 100 cells were then counted under the optical microscope (1000 x).

In the group of 50 strains isolated, 10 proved better able than the others to adhere to the cells used. As shown in Table 2, the strain with the greatest adherence capacity was *L. acidophilus* EUVAG.

### Hydrophobicity

The surface hydrophobicity possessed by some bacteria is a pre-requisite for the colonisation of the host epithelium.

The hydrophobicity of lactobacilli was evaluated by the Salt Aggregation Test, consisting of bacterial aggregation reactions at scalar concentrations of ammonium sulphate.

The lowest concentration of ammonium sulphate able to produce visible aggregation of the bacterial suspension was used as the hydrophobicity index.

Table 3 shows the high degree of hydrophobicity found in the 10 lactobacilli with the greatest adherence capacity, including *L. acidophilus* EUVAG.

### Effect of pH on growth

Stability under non-optimum environmental conditions plays an essential part among the minimum requirements that a probiotic must possess. Stability in an acid environment is particularly important because, if inoculated, the bacteria must be able to survive the acidity in order to reach some mucous membranes in viable conditions, and in any event must survive and colonise ecosystems, like the vaginal ecosystem, in which pH values not ideal for its growth may be present.

The tests were performed for all the lactobacilli the Applicant examined. The bacteria were inoculated into MRS broth at pH 3 (obtained by adding 1N HCl), pH 5 (normal value of broth) and pH 8 (obtained by adding 1N KOH).

The samples were incubated at 37°C in an atmosphere enriched with 5% CO₂, and at different intervals (0, 3 and 6 hours) a known inoculum was seeded on MRS agar in triplicate and incubated for 18 h so that a bacteria count could be conducted. The values thus obtained were converted to logarithms, and for the 3 values obtained at each time a mean value was calculated, and used for the graphs.

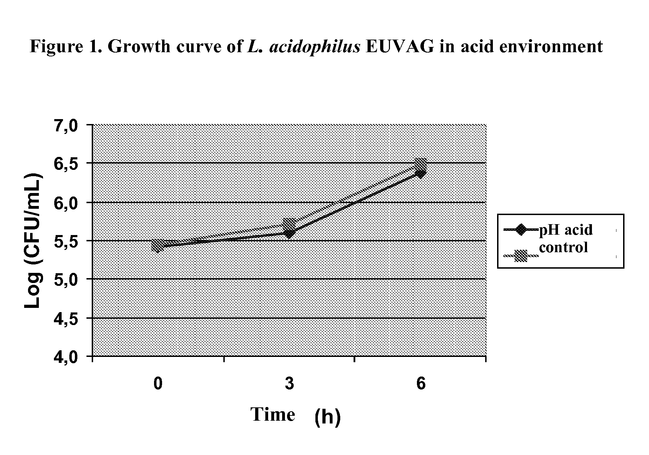

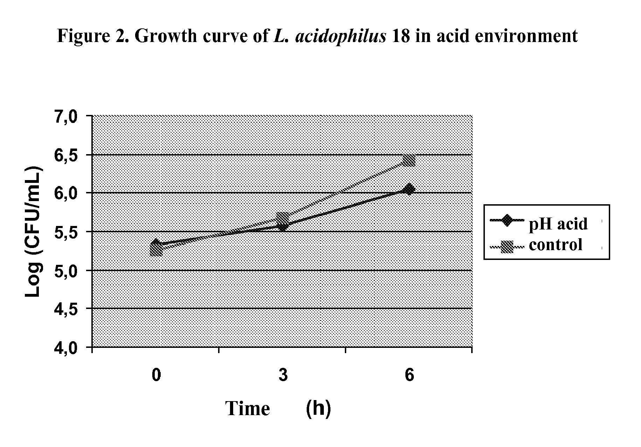

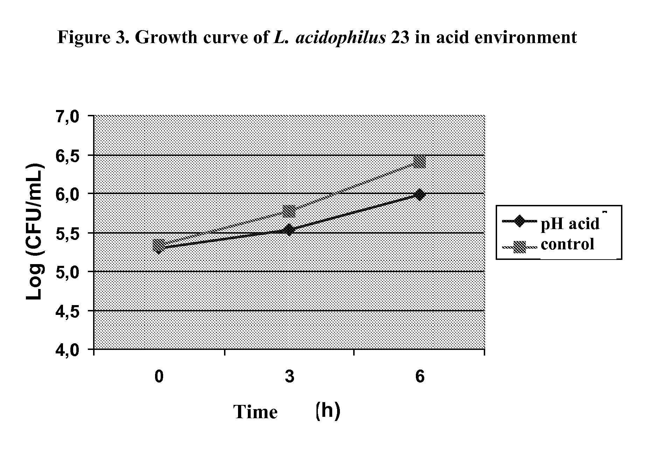

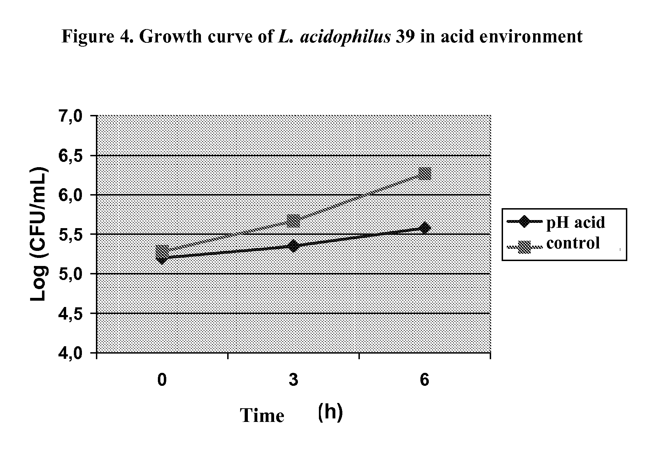

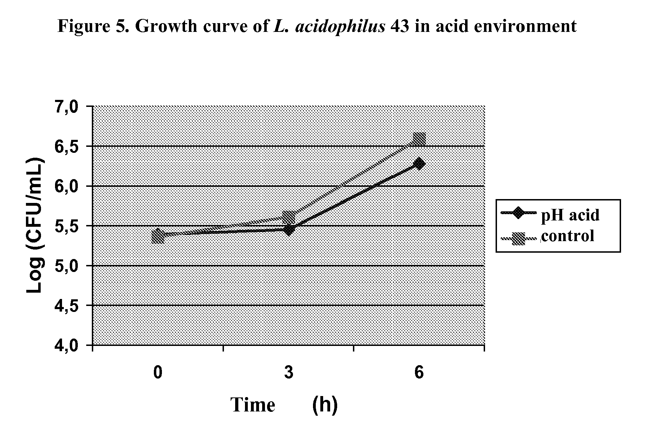

Figures 1-5 show the growth curves of 5 strains at acid pH compared with the growth observed in the same strain under optimum conditions (control curve).

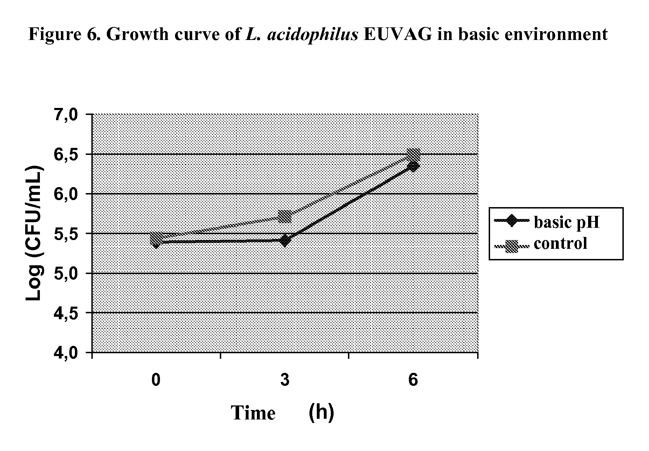

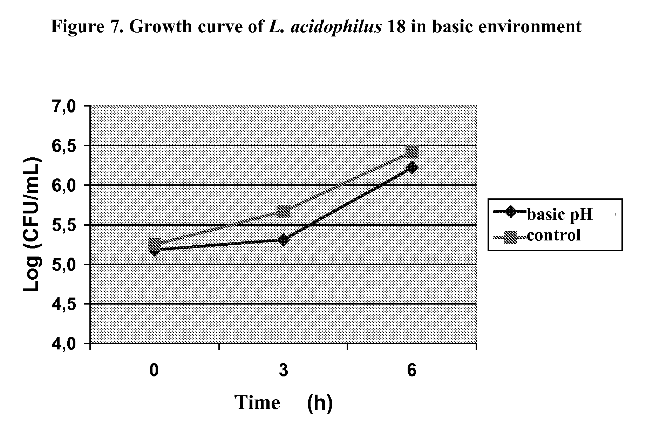

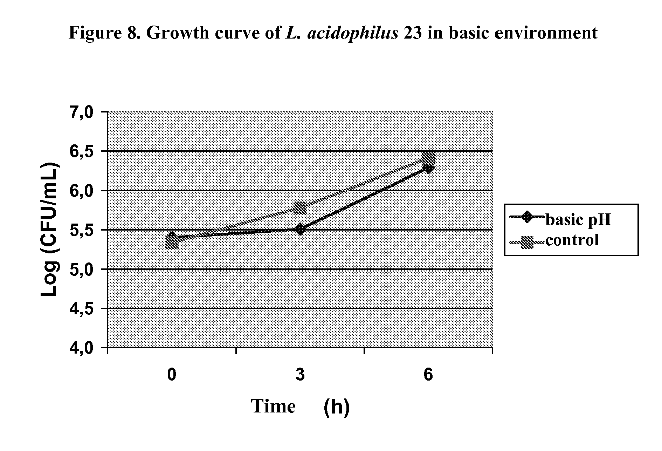

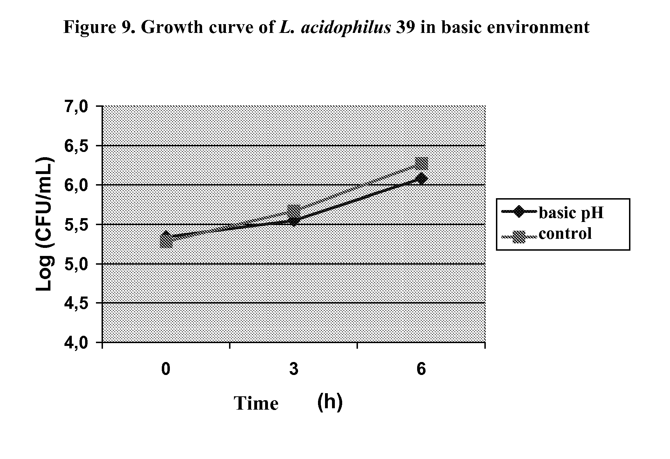

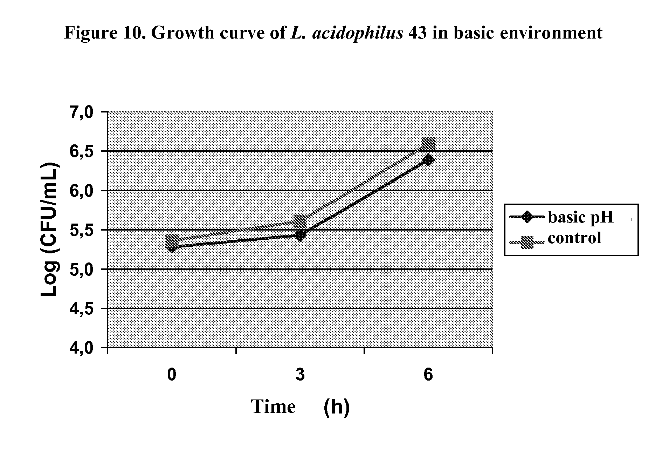

Figures 6-10 show the growth curves of the same 5 strains at a basic pH, compared with the corresponding growth controls.

They demonstrate that the growth of *L. acidophilus* EUVAG did not undergo any interference even under unfavourable pH conditions, unlike the other strains.

Tests conducted during Phase II demonstrate that of the 50 strains of *Lactobacillus* isolated, only 5 had the required probiotic characteristics (good adhesive properties and resistance to a non-optimum environment).

In particular, on the basis of a comparative analysis, the strain *L*. *acidophilus* EUVAG proved to possess better microbiological and adaptation characteristics than all the others.

In particular, its adhesive capacity and stability in non-optimum pH conditions indicate that *L. acidophilus* EUVAG is an ideal strain for the objectives in question.

### PHASE III

### Growth rate

For the lactobacilli the Applicant selected, the growth rate over time after inoculation into MRS broth was evaluated.

In particular, a known aliquot of the initial culture broth was seeded in MRS agar at regular intervals (0, 2, 4, 6, 8, 12 and 24 hours).

After 48 hours' incubation at 37°C in an atmosphere enriched with 5% CO₂, the bacteria present were counted.

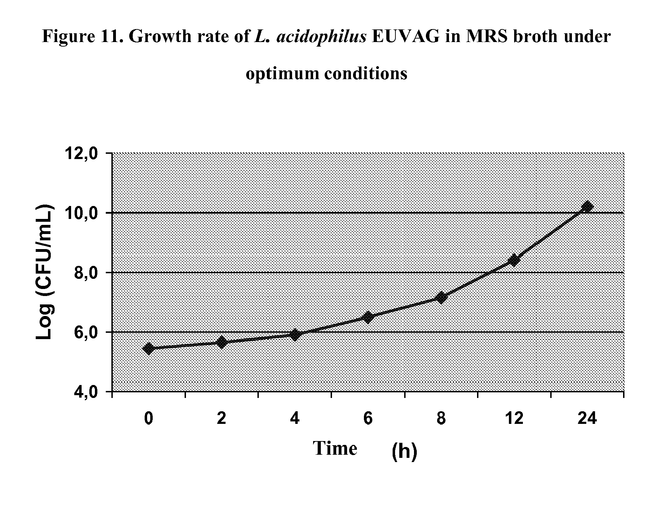

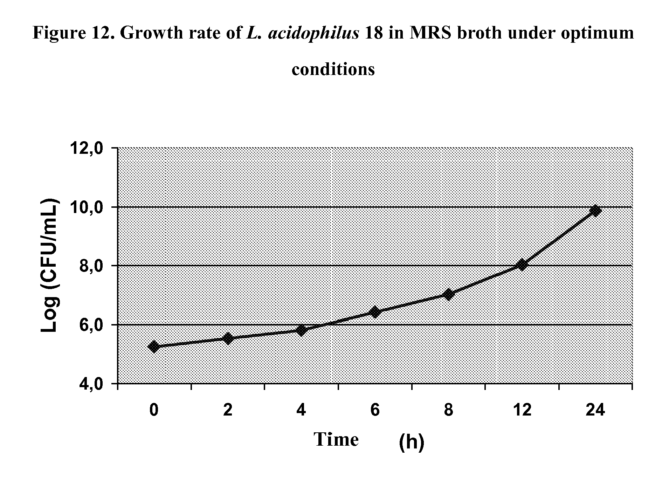

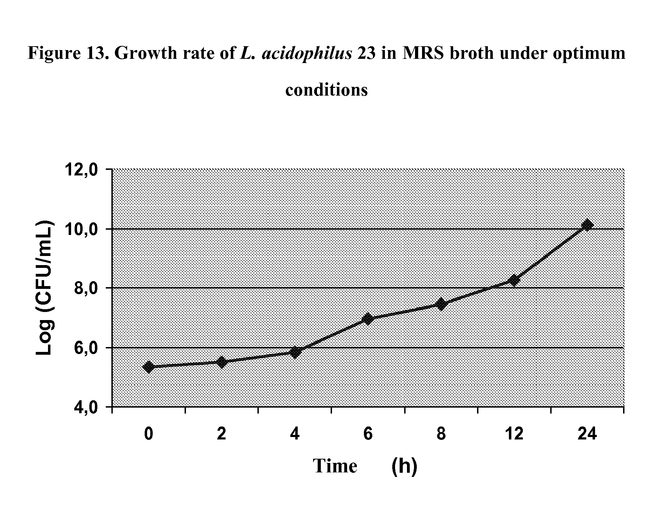

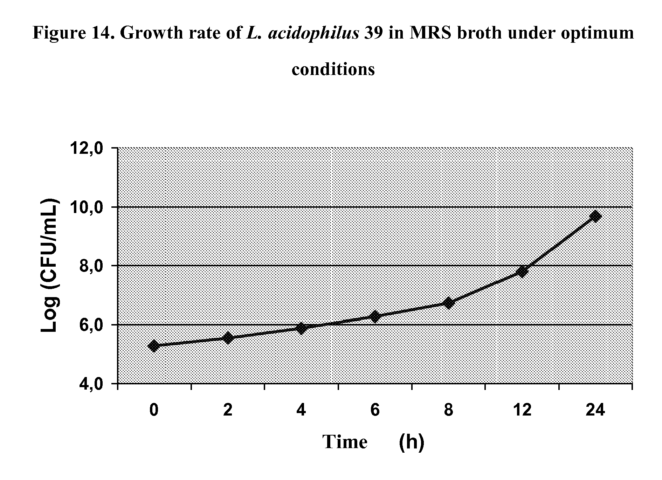

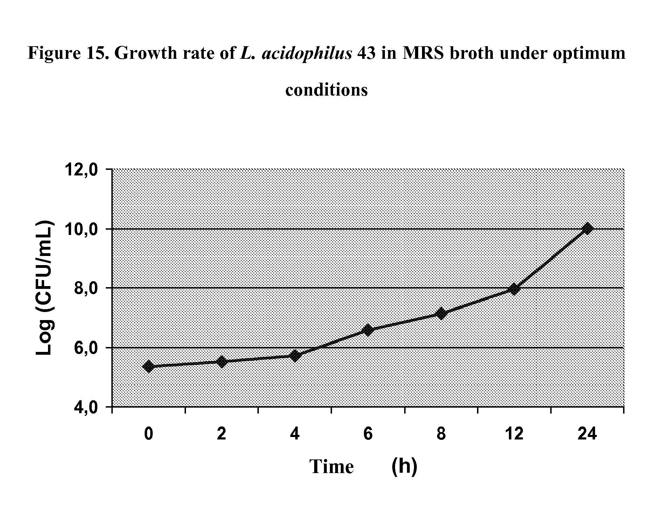

Figures 11-15 show the 24-hour growth curves of the strains previously selected by the Applicant.

As can be seen, the slope of the regression line, obtained from the mean values of the bacteria count in the unit of time, is greater in *L. acidophilus* EUVAG than the other lactobacilli.

### Resistance to freeze-drying

The *L. acidophilus* strains were freeze-dried (LabConco - Analytical Control freeze dryer) from a starting concentration of 10⁹ CFU/ml.

The protection used for the freeze-drying was based on low-fat milk and saccharose.

The bacteria count of the lyophilisate was then determined, after seeding in MRS agar, to evaluate its resistance to the freeze-drying process.

Of the 5 strains selected as described in point II, *L. acidophilus* EUVAG demonstrated optimum values of resistance and viability after freeze-drying (Table 6).

### PHASE IV

### Production of hydrogen peroxide

The production of hydrogen peroxide by lactobacilli is generally considered to be an important property for performing their antibacterial action. However, the real significance of this product as regards the antibacterial activity of lactobacilli requires further study, as it has been demonstrated that some lactobacilli which are currently successfully used as probiotics are unable to produce hydrogen peroxide.

The production of H₂O₂ was determined qualitatively on TMB (tetramethyl benzidine) plates containing Brucella agar with the addition of horseradish peroxidase, starch, haemin and vitamin K1. Serial dilutions from an overnight culture of *L. acidophilus* EUVAG were seeded on TMB medium. After anaerobic incubation for 72 hours at 37°C, the plates were exposed to air so that the identification reaction could take place. The production of H₂O₂ was detectable by the blue colour taken on by the colonies.

Using the method described, *L. acidophilus* EUVAG tested positive for hydrogen peroxide production.

### Production of lactic acid

The production of lactic acid by lactobacillary flora is considered to be the factor mainly responsible for the relative acidity of the vaginal environment.

The production of lactic acid in forms D and L was determined by enzyme assay (D-Lactic Acid/L-Lactic Acid, Boehringer Mannheim) on the supernatant obtained from the growth of *L. acidophilus* EUVAG for 24 h in MRS broth at 37°C. After centrifugation to obtain the supernatant, further enzyme reactions were inhibited by heating at 80°C for 15 min, followed by enzymatic determination. This is based on the conversion reaction of lactic acid to pyruvate, catalysed by the enzyme lactic dehydrogenase which leads to the simultaneous formation of NADH, the production of which is measured spectrophotometrically (UV 340 nm).

This method allowed a qualitative estimate of the production of lactic acid by *L. acidophilus* EUVAG. In fact, fairly large amounts of lactic acid of isomers L and D was found in the growth broth of the EUVAG strain obtained after 24 hours' incubation.

From the results obtained and those reported in the literature relating to lactobacilli it can be stated that: *L. acidophilus* EUVAG possesses properties which suggest its use in all forms of dysbiosis and variations of vaginal pH which can cause recurrent bacterial or fungal vaginal infections.

The compositions according to the invention, which are administered vaginally, will be prepared according to conventional methods well known in pharmaceutical technology, such as those described in "Remington's Pharmaceutical Handbook", Mack Publishing Co., N.Y., USA, together with suitable pharmaceutically acceptable excipients. Examples of these formulations are vaginal tablets, vaginal capsules and pessaries containing the lactobacillus in question in freeze-dried form. Said formulations will contain the lactobacillus in question in concentrations of not less than 10⁹ CFU/g of the product, for daily administration.

**Table 1 - Biochemical profile of L. acidophilus EUVAG**

| **Substrate** | **Fermentation** | **Substrate** | **Fermentation** |
|---|---|---|---|
| Glycerol | No | Esculin | No |
| Erythritol | No | Salicin | No |
| D-Arabinose | No | Cellobiose | Yes |
| L-Arabinose | No | Maltose | Yes |
| Ribose | No | Lactose | No |
| D- xylose | No | Melibiose | No |
| L-xylose | No | Saccharose | Yes |
| Adonitol | No | Trehalose | No |
| β-Methyl-D-xyloside | No | Inulin | No |
| Galactose | Yes | Melezitose | No |
| Glucose | Yes | Raffinose | No |
| Fructose | Yes | Starch | No |
| Mannose | Yes | Glycogen | No |
| Sorbose | No | Xylitol | No |
| Ramnose | No | Gentiobiose | No |
| Dulcitol | No | D-Turanose | No |
| Inositol | No | D-Lyxose | No |
| Mannitol | No | D-Tagatose | No |
| Sorbitol | No | D-Fucose | No |
| α-Methyl-D-mannoside | No | L-Fucose | No |
| α-Methyl-D-glucoside | No | D-Arabitol | No |
| N-Acetylglucosamine | Yes | L-Arabitol | No |
| | | Gluconate | No |
| Amygdalin | No | 2-Keto-gluconate | No |
| Arbutin | No | 5-Keto-gluconate | No |

**Table 2 - Adherence in vitro of 10 vaginally isolated lactobacilli to buccal cells**

| Lactobacilli | No. of bacteria/cell |
|---|---|
| *L. acidophilus EUVA G* | 54 ± 9.8 |
| *L. acidophilus VAG 3* | 38 ± 8.6 |
| *L. acidophilus VAG 11* | 41 ± 12.4 |
| *L. acidophilus VAG 18* | 49 ± 9.5 |
| *L. acidophilus VAG 22* | 24 ± 6.9 |
| *L. acidophilus VAG 23* | 50 ± 9.9 |
| *L. acidophilus VAG 32* | 44 ± 8.4 |
| *L. acidophilus VAG 39* | 51 ± 9.4 |
| *L. acidophilus VAG 43* | 34 ± 7.9 |
| *L. acidophilus VAG 48* | 55 ± 12.5 |

The adherence values are expressed as the mean number of adhering bacteria per cell ± standard deviation.

**Table 3 - Adherence in vitro of 10 vaginally isolated lactobacilli to Caco-2 cells**

| Lactobacilli | Caco-2 |
|---|---|
| *L. acidophilus EUVA G* | 62 ± 13.6 |
| *L. acidophilus VAG 3* | 49 ± 9.9 |
| *L. acidophilus VAG 11* | 47 ± 8.6 |
| *L. acidophilus VAG 18* | 53 ± 15.9 |
| *L. acidophilus VAG 22* | 26 ± 7.9 |
| *L. acidophilus VAG 23* | 54 ± 10.1 |
| *L. acidophilus VAG 32* | 49 ± 10.6 |
| *L. acidophilus VAG 39* | 57 ± 12.7 |
| *L. acidophilus VAG 43* | 41 ± 8.9 |
| *L. acidophilus VAG 48* | 52 ± 13.1 |

The adherence values are expressed as the mean number of adhering bacteria per cell ± standard deviation.

**Table 4 - Adherence in vitro of 10 vaginally isolated lactobacilli to HeLa cells**

| Lactobacilli | No. of bacteria/cell |
|---|---|
| *L. acidophilus EUVA G* | 65 ± 15.2 |
| *L. acidophilus VAG 3* | 49 ± 10.9 |
| *L. acidophilus VAG 11* | 45 ± 11.2 |
| *L. acidophilus VAG 18* | 50 ± 12.7 |
| *L. acidophilus VAG 22* | 29 ± 9.3 |
| *L. acidophilus VAG 23* | 52 ± 10.3 |
| *L. acidophilus VAG 32* | 53 ± 11.4 |
| *L. acidophilus VAG 39* | 61± 14.9 |
| *L. acidophilus VAG 43* | 39 ± 9.8 |
| *L. acidophilus VAG 48* | 55 ± 16.2 |

The adherence values are expressed as the mean number of adhering bacteria per cell ± standard deviation.

**Table 5 - Surface hydrophobicity of 10 vaginally isolated lactobacilli**

| **Lactobacilli** | **Ammonium sulphate concentration** |
|---|---|
| **L. acidophilus EUVAG** | 0.4 M |
| ***L. acidophilus VAG 3*** | 0.4 M |
| ***L. acidophilus VAG 11*** | 0.2 M |
| ***L. acidophilus VAG 18*** | 0.8 M |
| ***L. acidophilus VAG 22*** | 0.2 M |
| ***L. acidophilus VAG 23*** | 0.4 M |
| ***L. acidophilus VAG 32*** | 0.8 M |
| ***L. acidophilus VAG 39*** | 0.4 M |
| ***L. acidophilus VAG 43*** | 0.2 M |
| ***L. acidophilus VAG 48*** | 0.8 M |

**Table 6 - Resistance to freeze-drying**

| ***Evaluation of viability of 5 vaginally isolated lactobacillus strains after freeze-drying*** | | |
|---|---|---|
| **Lactobacilli** | **Log CFU/g** | **% survival** |
| ***L. acidophilus EUVA G*** | 8.82 | 62 |
| ***L. acidophilus VAG 18*** | 8.39 | 57 |
| ***L. acidophilus VAG 23*** | 8.20 | 52 |
| ***L. acidophilus VAG 39*** | 8.11 | 49 |
| ***L. acidophilus VAG 43*** | 8.08 | 46 |

Survival after freeze-drying is expressed as the percentage ratio between the bacteria count before freeze-drying and the bacteria count at the end of the freeze-drying process.

### REFERENCES

Akiyoshi H, Kazuto Y, Fumisaburo T: Isolation and characterization of an inhibitory substance against Escherichia coli produced by Lactobacillus acidophilus. Milchwissenschaft, 32: 727-30; 1977.
Bottazzi V, Friend BA, Shahani KM: Proprietà antitumorali dei batteri lattici e degli alimenti fermentati con batteri lattici. Il Latte, 10: 873-9; 1985.
Binder HJ, Filburn B, Floch M: Bile acid inhibition of intestinal anaerobic organisms. Am J Clin Nutr, 28: 119-25; 1975.
Burton JP, Cadieux P, Reid G: Improved understanding of the bacterial vaginal microflora of women before and after probiotic instillation. Appl Environ Microbiol 69: 97-101; 2003.
Conway P.L., Gorbach S.L., Goldin B.R. Survival of lactic acid bacteria in the human stomach and adhesion to intestinal cells. J Dairy Sci, 70: 1-12; 1987.
Drago L, Gismondo MR, Lombardi A, De Haen C, Gozzini L: Inhibition of in vitro growth of enteropathogens by novel lactobacillus strains of human intestinal origin. FEMS Microbiol Lett, 153: 455-63; 1997.
Drasar B.S., Hill M.J. Human intestinal flora. Academic Press, London, 1974.
Eschembach DA. History and review of bacterial vaginosis. Am J Obstet Gynecol, 169: 441-5; 1993
Eschenbach DA, Davick PR, Williams BL, Klebanoff SJ, Young-Smith K, Critclow CM, Holmes KK: Prevalence of hydrogen peroxide-producing Lactobacillus species in normal women and women with bacterial vaginosis. J Clin Microbiol, 27: 251-6; 1989.
Forsum U, Holst E, Larsson PG, Vasquez A, Jakobsson T, Mattsby-Baltzer I. Bacterial vaginosis - a microbiological and immunological enigma. APMIS, 113: 81-90; 2005
Gilliland SE, Speck GF, Nanyok GF, Giesbrecht FG: Influence of consuming non fermented milk containing L. acidophilus on fecal flora of healthy males. J Dairy Sci 61: 1-10; 1978.
Giorgi A, Torriani S, Dellaglio F, Bo G, Stola E, Bernuzzi L: Identification of vaginal lactobacilli from asymptomatic women. Microbiologica, 10: 377-84; 1987.
Gismondo M.R., Drago L., Lombardi A., Fassina C: An experimental model to reproduce some bacterial intestinal cocultures in germ-free mice. Drugs Exptl Clin Res XX, 149-52; 1994.
Gumma A., Filthuth I., Mirimanoff A: Etude de quelques procédés galéniques appliqués à la thérapeutique de substitution par Lactobacillus acidophilus. Pharmaceutica Acta Helvetiae 47: 164-7; 1979.
Hillier SL, Krohn MA, Rabe LK, Klebanoff SJ, Eschenbach DA: The normal vaginal flora, H2O2-producing lactobacilli, and bacterial vaginosis in pregnant women. Clin Infect Dis 16: S273-81; 1993
Johnson M.C., Ray B., Bhowmik T: Selection of Lactobacillus acidophilus strains for use in "acidophilus products". Antoine van Leeuwenhoek 53: 215-231, 1987.
Lee A. Neglected Niches: The microbial ecology of the gastrointestinal tract. In: Advances in Microbial Ecology 8,115-162, ed. Marshall K.C., Plenum Press N.Y., 1985.
Mardh P, Soltesz LV: In vitro interactions between Lactobacilli and other microrganisms occurring in the vaginal flora. Scand J Infect Dis Suppl, 40: 47-51, 1983.
May AM, Hawes SE, Hillier SL: The identification of vaginal Lactobacillus species and the demographic and microbiologic characteristics of women colonized by these species. J Infect Dis, 180: 1950-6; 1999.
Marelli G, Papaleo E, Ferrari A: Lactobacilli for prevention of urogenital infections: a review. Eur Rev Med Pharmacol Sci, 8: 87-95; 2004.
Melis GB, Ibba MT, Steri B, Kotsonis P, Matta V, Paoletti AM: Role of pH as a regulator of vaginal physiological environment. Merva Gynecol, 52: 111-21; 2000.
Ozkinay E, Terek MC, Yayci M, Kaiser R, Grob P, Tuncay G. The effectiveness of live lactobacilli in combination with low dose oestriol (Gynoflor) to restore the vaginal flora after treatment of vaginal infections. BJOG, 112: 234-40; 2005.
Redondo-Lopez V, Cook RL, Sobel JD: Emerging role of lactobacilli in the control and maintenance of the vaginal bacterial microflora. Rev Infect Dis, 12: 856-72; 1990.
Reid G, Bocking A: The potential for probiotics to prevent bacterial vaginosis and preterm labor. Am J Obstet Gynecol, 189: 1202-8.
Reid G, Bruce AW: Urogenital infections in women: can probiotics help? Postgrad Med J, 79: 428-32; 2003.
Silva M., Jacobus N.V., Deneke C., Gorbach S.L. Antimicrobial Substance from a human Lactobacillus strain. Antimicrob Agents Chemother 31, 1231-1233; 1987.
Van der Waij D. The colonization of the digestive tract in experimental animals and its consequence for infection prevention. New criteria for Antimicrobial Therapy. Ed. Excerpta Medica, Utrecht, 1979.
Wadstrom T, Andersson K, Sydow M, Axelsson L, Lindgren L, Gullmar B: Surface properties of lactobacilli isolated from the small intestine of pigs. J Applied Bacteriol, 62: 513-520; 1987.
Wood JR, Sweet RL, Catena A, Hadley WK, Robbie M. In vitro adherence of Lactobacillus species to vaginal epithelial cells. Am J Obstet Gynecol 153: 740-3; 1985.

## Claims

1. *Lactobacillus acidophilus* EUVAG strain deposited in the DSMZ collection under the number 19290 on 19 April 2007.

2. Compositions containing the *Lactobacillus acidophilus* strain claimed in claim 1, mixed with a compatible vehicle.

3. Compositions as claimed in claim 2, wherein the strain is present in freeze-dried form.

4. Compositions as claimed in either of claims 2 or 3, in the form of capsules, drinkable solutions or suspensions, or sachets of powder.

5. Compositions as claimed in any of claims 2 to 4, containing 10⁶ to 10¹⁰ cells of the strain per unit dose.

6. Use of the *Lactobacillus acidophilus* EUVAG strain for the preparation of medicaments useful for the treatment or prophylaxis of forms of dysbiosis and variations in vaginal pH which can cause recurrent bacterial or fungal vaginal infections.

7. Use as claimed in claim 6, for the preparation of medicaments for the treatment or prophylaxis of forms of dysbiosis and variations in vaginal pH which can cause recurrent bacterial or fungal vaginal infections.
